# EUROPÄISCHE PATENTSCHRIFT

(11) **EP 1 791 533 B1**
(45) Veröffentlichungstag und Bekanntmachung des Hinweises auf die Patenterteilung: **06.03.2013**
(21) Anmeldenummer: 05777179.2
(22) Anmeldetag: 06.09.2005
(51) Int. Cl.: A61K 9/70, C08K 3/34

(54) **TRANSDERMALES THERAPEUTISCHES SYSTEM MIT EINER HAFTSCHICHT, VERFAHREN ZUM SILIKONISIEREN EINER RÜCKSCHICHT DES SYSTEMS UND VERWENDUNG DER RÜCKSCHICHT**
TRANSDERMAL THERAPEUTIC SYSTEM COMPRISING AN ADHESIVE LAYER METHOD FOR SILICONIZING THE BACK LAYER OF THE SYSTEM AND USE OF SAID BACK LAYER
SYSTEME THERAPEUTIQUE TRANSDERMIQUE A COUCHE ADHESIVE, PROCEDE POUR LE SILICONAGE D'UNE COUCHE ARRIERE DE CE SYSTEME ET UTILISATION DE CETTE COUCHE ARRIERE

(30) Priorität: 13.09.2004 DE 102004044578
(43) Veröffentlichungstag der Anmeldung: 06.06.2007
(60) Teilanmeldung: 12006147.8
(73) Patentinhaber: LTS LOHMANN Therapie-Systeme AG, 56626 Andernach (DE)
(72) Erfinder: MÜLLER, Walter, 56626 Andernach (DE); LEONHARD, Johannes, 56170 Bendorf (DE)
(74) Vertreter: Zounek, Nikolai
(86) Internationale Anmeldenummer: PCT/EP2005/009547
(87) Internationale Veröffentlichungsnummer: WO 2006/029740

(56) Entgegenhaltungen:
- WO-A-01/74338
- WO-A-99/11265
- WO-A-2004/069286
- WO-A-2004/089361
- US-A1- 2004 057 985

## Beschreibung

Die Erfindung betrifft ein transdermales therapeutisches System enthaltend eine Rückschicht, ein e mit der Rückschicht in Kontakt stehende Polymerschicht, die Silikonklebstoffe aufweist und eine mit der Polymerschicht in Kontakt befindliche, ablösbare Schutzschicht, ein Verfahren zur Herstellung einer silikonisierten Rückschicht des Systems und die Verwendung der Rückschicht.

Transdermale therapeutische Systeme (TTS) bzw. wirkstoffhaltige Pflaster sind mittlerweile eine etablierte Arzneiform geworden. Trotzdem sind einige mit dieser Arzneiform verbundene Probleme bis heute nicht zufrieden stellend gelöst. Eines dieser Probleme betrifft speziell die so genannten Matrixsysteme bzw. Systeme, die einen den Matrixsystemen verwandten Aufbau haben. Ein solches Matrixsystem oder Matrix-TTS besteht im einfachsten Fall aus einer Rückschicht, einer wirkstoffhaltigen bevorzugt selbstklebenden Matrixschicht und einer vor Gebrauch zu entfernenden Schutzschicht. Oftmals wird beim Tragen der TTS nach einiger Zeit die Bildung eines mehr oder weniger schwachen dunklen Randes um das Pflaster auf der Haut beobachtet bzw. es bleiben beim Entfernen des TTS Klebstoffrückstände auf der Haut zurück. Besonders auffällig ist dieses Phänomen bei für die mehrtägige Anwendung geeigneten TTS zu beobachten. Ursache für beide Phänomene ist eine unzureichende Haftung der Pflastermatrix an der Rückschicht des Systems. Durch diese unzureichende Haftung und Körperbewegungen am Applikationsort wird ein Austreten des Klebstoffes an den Rändern des Systems verursacht und der ausgetretene Klebstoff kann in Kontakt mit der Kleidung kommen. Durch den Kontakt mit der Kleidung bleiben Stoffasern am ausgetretenen Klebstoff hängen und geben ihm meistens ein dunkles Aussehen. Nach dem Entfernen des TTS bleibt der ausgetretene Klebstoff in Form von dunklen Markierungen auf der Haut zurück. Ist die Haftung an der Rückschicht besonders schlecht, kann sich die Matrix auch flächenhaft von der Rückschicht lösen und auf der Haut zurück bleiben. Besonders anfällig für solche Phänomene sind Klebstoffe auf der Basis von Silikonen. Dies liegt daran, dass Silikonklebstoffe sehr unpolar sind und deshalb relativ schlecht auf den mehr oder weniger polaren Oberflächen der meistens aus Polyethylenterephthalat (PET) bestehenden Rückschicht haften. Hinzu kommt noch, dass Silikonklebstoffe nur über eine geringe Kohäsion verfügen und deshalb eine besonders stark ausgeprägte Tendenz zum Austreten aus dem System haben. Silikonklebstoffe verhalten sich deshalb wie eine viskose Flüssigkeit und das Spreiten auf eine größere Fläche wird deshalb auch als "Kalter Fluss" bezeichnet.

Aufgabe der vorliegenden Erfindung ist es daher, die Haftung der Rückschicht in einem transdermalen therapeutischen System mit der wirkstoffhaltigen Polymerschicht, die zumindest einen Silikonklebstoff enthält, zu verbessern und das Austreten von Silikonklebstoff aus dem System weitgehend zu verhindern.

Diese Aufgabe wird erfindungsgemäß durch ein eingangs beschriebenes transdermales therapeutisches System in der Weise gelöst, dass die Kontaktseite der Rückschicht mit einer durch Silikonisierung erhaltenen Haftschicht ausgerüstet ist.

In Ausgestaltung der Erfindung ist die Haftschicht eine Organopolysiloxanschicht. Insbesondere enthält die Haftschicht Organopolysiloxane mit Vinylgruppen und Organopolysiloxane mit Si-H-Gruppen.

In bevorzugter Weise ist die Rückschicht eine Kunststoff-Folie ausgewählt aus der Gruppe enthaltend Polyester, insbesondere Polyethylenterephthalat, Polypropylen, Polyethylen, Polyurethan, EVA-Schichten in Kombination mit Polyester, Polyvinylidenchlorid, Polyaramid Ethylen(meth)acrylat-Copolymere.

Die weitere Ausgestaltung der Erfindung ergibt sich aus den Merkmalen der Ansprüche 5 bis 9.

Das erfindungsgemäße Verfahren zur Herstellung einer Rückschicht, die in einem transdermalen therapeutischen System eingesetzt wird, zeichnet sich dadurch aus, dass die mit einer Polymerschicht, die mit Mikroreservoiren ausgerüstet ist, in Kontakt zu bringende Oberfläche der Rückschicht silikonisiert wird. Die geschieht bevorzugt in der Weise, dass Organopolysiloxane mit Vinylgruppen und Organopolysiloxane mit funktionellen Si-H-Gruppen gemischt werden und mit dem Gemisch in Gegenwart eines Katalysators die Oberfläche der Rückschicht beschichtet wird. Danach wird die beschichtete Oberfläche der Rückschicht bis zur Ausbildung einer auf der Rückschicht fest verankerten Organopolysiloxanschicht hitzebehandelt. Als Katalysator wird beispielsweise ein Platinkatalysator verwendet. Die Wärmebehandlung erfolgt in einem Wärmeofen oder in einem Wärmedurchlauftunnel. Die Temperatur beträgt etwa 80 bis 100 °C, kann aber auch unter 80 °C liegen.

Jedes selbstklebende System, sei es nun ein transdermales therapeutisches System, ein wirkstofffreies Pflaster, ein Etikett oder ein Klebeband muß vor Gebrauch durch eine wieder ablösbare Schutzschicht geschützt werden. Die Schutzschicht kann aus verschiedenen Materialien wie z.B. PET, Polyethylen oder Polypropylen bestehen und ist auf der mit dem Klebstoff in Kontakt kommenden Seite speziell behandelt, um sie möglichst leicht von der Klebstoffschicht ablösbar zu machen. Für die Verwendung in Kombination mit nicht auf Silikonen beruhenden Klebstoffen besteht diese Oberflächenbehandlung meistens aus einer so genannten Silikonisierung. Bei dieser Silikonisierung werden in einem Beschichtungsprozess z.B. Organopolysiloxane mit Vinylgruppen und Organopolysiloxane mit SiH-funktionellen Gruppen im Gemisch in Gegenwart eines Platinkatalysator auf die zu behandelnde Folie beschichtet wobei sich durch eine Hitzebehandlung eine auf dem Substrat fest haftende Organopolysiloxanschicht bildet. Während nicht auf Silikonen beruhende Klebstoffe, wie zum Beispiel Polyacrylatkleber, auf so behandelten Oberflächen außerordentlich schlecht binden, haften Silikonklebstoffe auf solchen Oberflächen extrem gut. In Trageversuchen zeigte es sich, dass wirkstofffreie Pflaster auf Basis von Silikonklebstoffen und einer dermaßen behandelten Rückschicht erheblich weniger Klebestoffrückstände nach dem Entfernen auf der Haut zurücklassen, und dass die Tendenz zur Bildung von "schwarzen" Rändern um das Pflaster erheblich reduziert ist.

Bei auf Silikonklebstoffen basierenden TTS wird die Permeationsrate des Wirkstoffs durch die Haut durch die externe Anwendung von Hitze am applizierten TTS gesteigert. Solche Systeme sind z.B. in der US 6,488,959 A1 und der US 6,261,595 A1 detailliert beschrieben. Durch die Temperatur, die dabei durchaus auf bis zu etwa 45 °C ansteigen kann, wird die Tendenz des Silikonklebstoffes zum Spreiten durch kalten Fluß massiv verstärkt Unter solchen Bedingungen ist vermehrt mit der Bildung von dunklen Rändern um das Pflaster und dem Verbleiben von Klebstoffesten auf der Haut nach dem Entfernen des Pflasters zu rechnen. In solchen Systemen ist eine Silikonisierung der Rückschicht von großem Vorteil, da trotz der Applikation von Wärme ein Spreiten des Klebstoffes weitgehend vermieden wird.

Als ebenfalls besonders wichtig erweist sich die Silikonisierung der Rückschicht in Verbindung mit so genannten Mikroreservoirsystemen auf Basis von Silikonklebstoffen und ambiphilen Lösemitteln für den Wirkstoff. Solche Mikroreservoirsysteme sind in der EP 1 191 927 B1 detailliert beschrieben. Bei der Herstellung von solchen Systemen wird der Wirkstoff in einem ambiphilen Lösemittel wie z.B. Dipropylenglycol oder 1,3-Butandiol gelöst und die Lösung in der Lösung des Klebstoffes dispergiert. Anschließend wird die Dispersion auf die Schutzschicht des späteren TTS beschichtet, das Lösemittel des Klebstoffes entfernt und der getrocknete Film mit der Rückschicht laminiert Es hat sich nun gezeigt, dass eine unsilikonisierte Rückschicht auf einem solchen Film praktisch nicht haftet. Ursache ist die Bildung eines sehr dünnen Films aus dem ambiphilen Lösemittel auf der Oberfläche des getrockneten wirkstoffhaltigen Films. Eine Änderung des Herstellverfahrens, z.B. eine Beschichtung direkt auf die Rückschicht, verbessert dabei die Haftung des getrockneten Films nicht. Eine silikonisierte Rückschicht jedoch ergibt, unabhängig von der chemischen Natur der Folie selbst, unmittelbar nach Kontakt mit einer mikroreservoirhaltigen Schicht eine sehr gute Haftung. Eine verbesserte Haftung kann zwar auch durch die Verwendung von weniger inerten Materialien, wie z.B. Copolymere aus Ethylen und Vinylacetat, für die Rückschicht von solchen Systemen erreicht werden, jedoch haben solche Materialien den Nachteil, dass sie neben dem Lösemittel auch Wirkstoff aufnehmen. Mikroreservoirsysteme wie oben beschrieben sind deshalb am besten unter Verwendung von silikonisierten Rückschichten aus Polyester oder ähnlich inerten Materialien realisierbar.

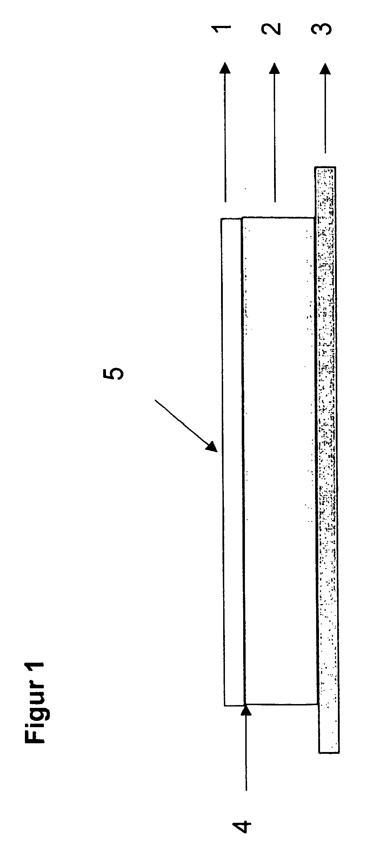

Die einzige Figur zeigt ein transdermales therapeutisches System 5, das eine Rückschicht 1, eine Haftschicht 4, die auf der Rückschicht fest verankert ist, eine Polymerschicht 2 und eine ablösbare Schutzschicht 3 umfasst. Die Haftschicht 4 ist eine Organopolysiloxanschicht, die Organopolysiloxane mit Vinylgruppen und Organopolysiloxane mit funktionellen Si-H-Gruppen enthält. Die Rückschicht 1 besteht aus einer Kunststoff-Folie. Geeignete Kunststoffe sind Polyester, insbesondere Polyethylenterephthalat, Polypropylen, Polyethylen, Polyurethan, EVA-Schichten in Kombination mit Polyester, PVDC, Polyaramide, Ethylen(meth)acrylat-Copolymere. Weitere geeignete Materialien sind Ethylencelluose, Celluloseacetat, Cellophan, Papier, Metall-Polymer-Verbund, Ethylenvinylacetat-Copolymere.

Die Silikonklebstoffe in der Polymerschicht 2 werden beispielsweise aus der Gruppe der Polysiloxane und Mischungen von Polysiloxanen ausgewählt.

In der Polymerschicht 2 sind Mikroreservoire vorhanden, die zumindest einen Wirkstoff enthalten, der in einem ambiphilen Lösemittel gelöst ist. Als ambiphile Lösemittel sind 1,3-Butandiol, Diethylenglykolmonomethylether, Diethylenglykoldimethylether, Dipropylenglykol, Propylenglykol, Tetrahydrofurfiuylalkohol, Diethylenglykolmonobutylether, Carbonsäureester von Tri- und Diethylengylkol, polyoxyethylierte Fettalkohole von 6 - 18 C-Atomen geeignet.

Die Schutzschicht 3 steht an beiden Seiten der Polymerschicht 2 über, so dass sie problemlos greifbar und ablösbar ist.

Auf die Herstellung von Mikroreservoirsystemen hat die Verwendung einer silikonisierten Rückschicht keinerlei Einfluss. Selbst bei schon vermarkteten TTS kann rein technisch die nicht silikonisierte Rückschicht ohne Probleme durch eine silikonisierte Rückschicht ausgetauscht werden. Die Eigenschaften des TTS bezüglich der Wirkstoffabgabe bleiben bei einem Austausch unverändert, da die aufgebrachte Silikonschicht mit weniger als 10 Mikrometern Dicke praktisch keinen Wirkstoff aufnimmt.

Zusammenfassend läßt sich somit sagen, dass durch die Verwendung von silikonisierten Rückschichten die Trageeigenschaften von auf Silikonklebstoffen basierenden TTS entscheidend verbessert werden, und spezielle Systeme, wie z.B. Mikroreservoirsysteme auf Basis von Silikonklebstoffen und ambiphilen Lösemitteln, erst technisch machbar werden.

## Patentansprüche

1. Transdermales therapeutisches System (5) enthaltend eine Rückschicht (1), eine mit der Rückschicht (1) in Kontakt stehende Polymerschicht (2), die Silikonklebstoffe aufweist und eine mit der Polymerschicht (2) in Kontakt befindliche, ablösbare Schutzschicht (3), **dadurch gekennzeichnet, dass** die Kontaktseite der Rückschicht (1) mit einer durch Silikonisierung erhaltenen Haftschicht (4) ausgerüstet ist, die fest mit der Rückschicht verankert ist und Organopolysiloxane mit Vinylgruppen und Organopolysiloxane mit Si-H-Gruppen enthält.

2. Transdermales therapeutisches System nach Anspruch 1, **dadurch gekennzeichnet, dass** die Rückschicht (1) eine Kunststoff-Folie, ausgewählt aus der Gruppe enthaltend Polyester, insbesondere Polyethylenterephthalat, Polypropylen, Polyethylen, Polyurethan, EVA-Schichten in Kombination mit Polyester, Polyvinylidenchlorid, Polyaramid, Ethylen(meth)acrylat-Copolymer, ist.

3. Transdermales therapeutisches System nach Anspruch 1, **dadurch gekennzeichnet, dass** die Rückschicht (1) aus einem Material besteht, ausgewählt aus der Gruppe enthaltend Ethylcellulose, Celluloseacetat, Cellophan, Papier, Metall-Polymerverbund, Ethylenvinylacetat-Copolymere.

4. Transdermales therapeutisches System nach Anspruch 1, **dadurch gekennzeichnet, dass** die Polymerschicht (2) einen Silikonklebstoff auf der Basis von Silikonpolymeren und Harz enthält.

5. Transdermales therapeutisches System nach Anspruch 1, **dadurch gekennzeichnet, dass** die Silikonklebstoffe aus der Gruppe Polysiloxane und Mischungen von Polysiloxanen ausgewählt sind.

6. Transdermales therapeutisches System nach Anspruch 1, **dadurch gekennzeichnet, dass** die Polymerschicht (2) Mikroreservoire für pharmazeutische Wirkstoffe, die in einem ambiphilen Lösemittel gelöst sind, enthält.

7. Transdermales therapeutisches System nach Anspruch 1, **dadurch gekennzeichnet, dass** das ambiphile Lösemittel aus der Gruppe enthaltend 1,3-Butandiol, Diethylenglykolmonoethylether, Diethylenglykoldimethylether, Dipropylenglykol, Propylenglykol, Tetrahydrofurfurylalkohol, Diethylenglykolmonobutylether, Carbonsäureester von Tri- und Diethylenglykol, polyoxyethylierte Fettalkohole von 6 - 18 C-Atomen oder Mischungen hiervon, ausgewählt ist.

8. Verfahren zur Herstellung einer Rückschicht, die in einem transdermalen therapeutischen System nach den Ansprüchen 1 bis 8 eingesetzt wird, **dadurch gekennzeichnet, dass** die mit einer Polymerschicht, die mit Mikroreservoiren ausgerüstet ist, in Kontakt zu bringende Oberfläche der Rückschicht silikonisiert wird, indem Organopolysiloxane mit Vinylgruppen und Organopolysiloxane mit funktionellen Si-H-Gruppen gemischt werden und mit dem Gemisch in Gegenwart eines Katalysators unter Hitzeanwendung die Oberfläche der Rückschicht beschichtet wird.

9. Verfahren nach Anspruch 8, **dadurch gekennzeichnet, dass** die beschichtete Oberfläche der Rückschicht bis zur Ausbildung einer auf der Rückschicht fest verankerten Organopolysiloxanschicht hitzebehandelt wird.

10. Verwendung einer silikonisierten Rückschicht in einem transdermalen therapeutischen System nach den Ansprüchen 1 bis 7.

11. Verwendung einer Rückschicht nach Anspruch 10, auf der durch Silikonisierung eine Organopolysiloxanschicht fest verankert ist.

12. Verwendung einer Rückschicht nach Anspruch 11, wobei eine Polymerschicht des Systems die Mikroreservoire und mindestens einen darin gelösten Wirkstoff sowie zumindest einen Silikonklebstoff enthält, mit der Organopolysiloxanschicht selbstklebend verbunden ist.

## Claims

1. A transdermal therapeutic system (5) comprising a backing layer (1), a polymer layer (2) in contact with the backing layer (1) and comprising silicone adhesives, and a detachable protective layer (3) in contact with the polymer layer (2), wherein the contact face of the backing layer (1) has been provided with an adhesive layer (4) obtained by siliconization which is firmly anchored to the backing layer and comprises organopolysiloxanes containing vinyl groups and organopolysiloxanes containing Si-H groups.

2. The transdermal therapeutic system as claimed in claim 1, wherein the backing layer (1) is a polymeric film material selected from the group consisting of polyesters, especially polyethylene terephthalate, polypropylene, polyethylene, polyurethane, EVA layers in combination with polyester, polyvinylidene chloride, polyaramids, and ethylene-(meth)acrylate copolymer.

3. The transdermal therapeutic system as claimed in claim 1, wherein the backing layer (1) is composed of a material selected from the group consisting of ethylcellulose, cellulose acetate, cellophane, paper, metal-polymer composite, and ethylene-vinyl acetate copolymers.

4. The transdermal therapeutic system as claimed in claim 1, wherein the polymer layer (2) comprises a silicone adhesive based on silicone polymers and resin.

5. The transdermal therapeutic system as claimed in claim 1, wherein the silicone adhesives are selected from the group consisting of polysiloxanes and polysiloxane mixtures.

6. The transdermal therapeutic system as claimed in claim 1, wherein the polymer layer (2) comprises microreservoirs for active pharmaceutical ingredients which are in solution in an ambiphilic solvent.

7. The transdermal therapeutic system as claimed in claim 1, wherein the ambiphilic solvent is selected from the group consisting of 1,3-butanediol, diethylene glycol monoethyl ether, diethylene glycol dimethyl ether, dipropylene glycol, propylene glycol, tetrahydrofurfuryl alcohol, diethylene glycol monobutyl ether, carboxylic esters of tri- and diethylene glycol, polyoxyethylated fatty alcohols of 6-18 carbon atoms, and mixtures thereof.

8. A method of producing a backing layer used in a transdermal therapeutic system as claimed in claims 1 to 7, which comprises siliconizing the surface of the backing layer that is to be contacted with a polymer layer which has been provided with microreservoirs by mixing organopolysiloxanes containing vinyl groups and organopolysiloxanes containing functional Si-H groups and coating the surface of the backing layer with the mixture in the presence of a catalyst and with the application of heat.

9. The method as claimed in claim 8, wherein the coated surface of the backing layer is heat-treated until an organopolysiloxane layer forms that is firmly anchored on the backing layer.

10. The use of a siliconized backing layer in a transdermal therapeutic system as claimed in claims 1 to 7.

11. The use of a backing layer, as claimed in claim 10, on which an organopolysiloxane layer is firmly anchored by siliconization.

12. The use of a backing layer, as claimed in claim 11, a polymer layer of the system, comprising microreservoirs and at least one active ingredient dissolved therein and also at least one silicone adhesive, being self-adhesively connected to the organopolysiloxane layer.

## Revendications

1. Système thérapeutique transdermique (5) comprenant une couche arrière (1), une couche polymère (2) en contact avec la couche arrière (1), qui présente des adhésifs en silicone et une couche de protection (3) détachable se trouvant en contact avec la couche polymère (2), **caractérisé en ce que** le côté contact de la couche arrière (1) est muni d'une couche adhésive (4) obtenue par siliconage, qui est ancrée de manière fixe à la couche arrière et comprend des organopolysiloxanes avec des groupes vinyle et des organopolysiloxanes avec des groupes Si-H.

2. Système thérapeutique transdermique selon la revendication 1, **caractérisé en ce que** la couche arrière (1) est un film plastique, sélectionné dans le groupe comprenant le polyester, en particulier le polyéthylène téréphtalate, le polypropylène, le polyéthylène, le polyuréthane, des couches d'EVA en association avec le polyester, le polychlorure de vinylidène, les polyaramides, le copolymère d'éthylène-(méth)acrylate.

3. Système thérapeutique transdermique selon la revendication 1, **caractérisé en ce que** la couche arrière (1) se compose d'un matériau sélectionné dans le groupe comprenant l'éthylcellulose, l'acétate de cellulose, le cellophane, le papier, un composite métal/polymère, les copolymères d'éthylène-acétate de vinyle.

4. Système thérapeutique transdermique selon la revendication 1, **caractérisé en ce que** la couche polymère (2) comprend un adhésif en silicone sur la base de polymères de silicone et de résine.

5. Système thérapeutique transdermique selon la revendication 1, **caractérisé en ce que** les adhésifs en silicone sont sélectionnés dans le groupe des polysiloxanes et des mélanges de polysiloxanes.

6. Système thérapeutique transdermique selon la revendication 1, **caractérisé en ce que** la couche polymère (2) comprend des microréservoirs pour agents pharmaceutiques qui sont dissous dans un solvant ambiphile.

7. Système thérapeutique transdermique selon la revendication 1, **caractérisé en ce que** le solvant ambiphile est sélectionné dans le groupe comprenant le 1,3-butanediol, l'éther monoéthylique de diéthylène glycol, l'éther diméthylique de diéthylène glycol, le dipropylène glycol, le propylène glycol, l'alcool tétrahydrofurfurylique, l'éther monobutylique de diéthylène glycol, l'ester d'acide carbonique du tri- et de diéthylène glycol, les alcools gras polyoxyéthylés de 6 à 18 atomes de C ou leurs mélanges.

8. Procédé de fabrication d'une couche arrière qui est mise en oeuvre dans un système thérapeutique transdermique selon l'une des revendications 1 à 8, **caractérisé en ce que** la surface de la couche arrière à mettre en contact avec une couche polymère qui est munie de microréservoirs est siliconée, **en ce que** des organopolysiloxanes avec des groupes vinyle et des organopolysiloxanes avec des groupes Si-H fonctionnels sont mélangés et la surface de la couche arrière est recouverte du mélange en présence d'un catalyseur par application de chaleur.

9. Procédé selon la revendication 8, **caractérisé en ce que** la surface recouverte de la couche arrière est traitée thermiquement jusqu'à la formation d'une couche d'organopolysiloxanes ancrée de manière fixe sur la couche arrière.

10. Utilisation d'une couche arrière siliconée dans un système thérapeutique transdermique selon l'une des revendications 1 à 7.

11. Utilisation d'une couche arrière selon la revendication 10, sur laquelle une couche d'organopolysiloxanes est ancrée de manière fixe par siliconage.

12. Utilisation d'une couche arrière selon la revendication 11, dans laquelle une couche polymère du système comprend les microréservoirs et au moins un agent dissous dedans ainsi qu'au moins un adhésif en silicone, avec laquelle la couche d'organopolysiloxanes est reliée de manière autoadhésive.
